# EUROPEAN PATENT SPECIFICATION

(11) **EP 3 950 615 B1**
(45) Date of publication and mention of the grant of the patent: **01.03.2023**
(21) Application number: 21189209.6
(22) Date of filing: 02.08.2021
(51) Int. Cl.: C03C 3/062, C03C 3/093, C03C 3/087, C03C 3/095, C03C 3/097, C03C 4/00, C03C 8/04

(54) **DENTAL COMPOSITION**
DENTALZUSAMMENSETZUNG
COMPOSITION DENTAIRE

(30) Priority: 04.08.2020 JP 2020132351
(43) Date of publication of application: 09.02.2022
(73) Proprietor: GC Corporation, Sunto-gun, Shizuoka 410-1307 (JP)
(72) Inventor: FUJIMOTO, Ayaka, Tokyo, 174-8585 (JP); MIYAKE, Takahiro, Tokyo, 174-8585 (JP); YOSHIMITSU, Ryosuke, Tokyo, 174-8585 (JP); SHIDA, Enzo, Tokyo, 174-8585 (JP); TAKAHASHI, Toshihiro, Tokyo, 174-8585 (JP); NAGANO, Yasuyuki, Tokyo, 174-8585 (JP); TANAKA, Koji, Tokyo, 174-8585 (JP); HOKII, Yusuke, Tokyo, 174-8585 (JP); KATO, Katsuhito, Tokyo, 174-8585 (JP); MORI, Daizaburo, Tokyo, 174-8585 (JP)
(74) Representative: Müller-Boré & Partner Patentanwälte PartG mbB

(56) References cited:
- WO-A1-2005/115305
- WO-A1-2019/069564
- JP-A- 2019 034 891
- US-B1- 6 297 181

## Description

The present application is based on and claims priority to Japanese patent application No. 2020-132351 filed on August 4,2020, with the Japanese Patent Office

The disclosures herein relate to dental glass powders and dental compositions.

As a dental glass powder, aluminosilicate glass powders are well known. Among aluminosilicate glass powders, a fluoroaluminosilicate glass powder is widely used because the fluoroaluminosilicate glass powder is expected to exhibit the inhibition of dental decalcification (for example, see Patent Documents 1 and 2).

Conventionally, a fluoroaluminosilicate glass powder is used in glass ionomer cements.

A glass ionomer cement generally has a powder component including a fluoroaluminosilicate glass powder, a polycarboxylic acid polymer, and a liquid component including water.
Patent Document 1: Japanese Patent Laid-Open No. 62-67008
Patent Document 2: Japanese Unexamined Patent Laid-Open No. 63-201038

However, it is desired to improve the antibacterial effect, transparency, and inhibition of dental decalcification of a cured product of a glass ionomer cement.

One aspect of the invention is to provide a dental glass powder and a dental composition capable of improving the antibacterial effect, transparency, and inhibition of dental decalcification of a cured product of a glass ionomer cement.

One aspect of the invention is a dental glass powder includes 15 to 40% by mass of zinc oxide (ZnO), 20 to 55% by mass of silicon oxide (SiO₂), 6 to 20% by mass of aluminum oxide (Al₂O₃), 1 to 13% by mass of calcium oxide (CaO), and 1 to 19% by mass of fluorine (F).

The invention is defined in independent claim 1 and its associated dependent claims.

According to one aspect of the present invention, a dental glass powder and a dental composition capable of improving the antibacterial effect, transparency, and inhibition of dental decalcification of a cured product of a glass ionomer cement can be provided.

Next, an embodiment for carrying out the present invention will be described.

### <Dental Glass Powder>

A dental glass powder of the present embodiment includes zinc, silicon, aluminum, calcium, and fluorine.

The amount of zinc oxide (ZnO) in the dental glass powder of the present embodiment is 15 to 40% by mass and more preferably 20 to 30% by mass. When the amount of zinc oxide (ZnO) in the dental glass powder of the present embodiment is 15% by mass or more, the antibacterial effect of the cured product of the glass ionomer cement including the dental glass powder of the present embodiment is improved. When the amount of zinc oxide (ZnO) in the dental glass powder is 40% by mass or less, the transparency of the cured product of the glass ionomer cement including the dental glass powder of the present embodiment is improved.

The amount of silicon oxide (SiO₂) in the dental glass powder of the present embodiment is 20 to 55% by mass and more preferably 30 to 50% by mass. When the amount of silicon oxide (SiO₂) in the dental glass powder of the present embodiment is 20% by mass or more, the transparency of the cured product of the glass ionomer cement including the dental glass powder of the present embodiment is improved. When the amount of silicon oxide (SiO₂) in the dental glass powder is 55% by mass or less, the dental glass powder of the present embodiment is easily manufactured.

The amount of aluminum oxide (Al₂O₃) in the dental glass powder of the present embodiment is 6 to 20% by mass and more preferably 7 to 16% by mass. When the amount of aluminum oxide (Al₂O₃) in the dental glass powder of the present embodiment is 6% by mass or more, the transparency of the cured product of the glass ionomer cement including the dental glass powder of the present embodiment is improved. When the amount of aluminum oxide (Al₂O₃) in the dental glass powder is 20% by mass or less, the dental glass powder of the present embodiment is easily manufactured.

The amount of calcium in the dental glass powder of the present embodiment is 1 to 13% by mass and more preferably 5 to 13% by mass. When the amount of calcium oxide (CaO) in the dental glass powder of the present embodiment is 1% by mass or more, the inhibition of dental decalcification of the cured product of the glass ionomer cement including the dental glass powder of the present embodiment is improved. When the amount of calcium oxide (CaO) in the dental glass powder is 13% by mass or less, the dental glass powder of the present embodiment is easily manufactured.

The amount of fluorine (F) in the dental glass powder of the present embodiment is 1 to 19% by mass and more preferably 3 to 15% by mass. When the amount of fluorine (F) in the dental glass powder of the present embodiment is 1% by mass or more, the inhibition of dental decalcification of the cured product of the glass ionomer cement including the dental glass powder of the present embodiment is improved. When the amount of fluorine (F) in the dental glass powder according to the present embodiment is 19% by mass or less, the dental glass powder of the present embodiment is easily manufactured.

The dental glass powder of the present embodiment may further include boron, phosphorous, sodium, and the like.

The amount of boron oxide (B₂O₃) in the dental glass powder of the present embodiment is preferably 0 to 15% by mass and more preferably 0 to 10% by mass. The amount of boron oxide (B₂O₃) in the dental glass powder of the present embodiment may be more than 0% by mass and 15% by mass or less. When the amount of boron oxide (B₂O₃) in the dental glass powder of the present embodiment is 15% by mass or less, the dental glass powder of the present embodiment is easily manufactured.

The amount of phosphorus (V) oxide (P₂O₅) in the dental glass powder of the present embodiment is preferably 0 to 10% by mass and more preferably 0 to 7% by mass. The amount of phosphorus (V) oxide (P₂O₅) in the dental glass powder of the present embodiment may be more than 0% by mass and 10% by mass or less. When the amount of phosphorus (V) oxide (P₂O₅) in the dental glass powder of the present embodiment is 10% by mass or less, the workability of the glass ionomer cement including the dental glass powder of the present embodiment is improved.

The amount of sodium oxide (Na₂O) in the dental glass powder of the present embodiment is preferably 0 to 10% by mass and more preferably 1 to 5% by mass. The amount of sodium oxide (Na₂O) in the dental glass powder of the present embodiment may be more than 0% by mass and 10% by mass or less. When the amount of sodium oxide (Na₂O) in the dental glass powder of the present embodiment is 10% by mass or less, the dental glass powder of the present embodiment is easily manufactured.

An example of the composition of a dental glass powder of the present embodiment is described below.
ZnO: 15 to 40% by mass
SiO₂: 20 to 55% by mass
Al₂O₃: 6 to 20% by mass
CaO: 1 to 13% by mass
F: 1 to 19% by mass
B₂O₃: 0 to 10% by mass
P₂O₅: 0 to 10% by mass
Na₂O: 0 to 10% by mass

The number average particle size of the dental glass powder in the present embodiment is preferably 0.02 to 30 µm and more preferably 0.02 to 20 µm. When the number average particle size of the dental glass powder of the present embodiment is 0.02 µm or more, the workability of the glass ionomer cement including the dental glass powder of the present embodiment is improved. When the number average particle size is 30 µm or less, the wear resistance of the cured product of the glass ionomer cement including the dental glass powder of the present embodiment is improved.

### <Method of Manufacturing Dental Glass Powder>

The dental glass powder of the present embodiment can be manufactured by melting a raw material composition followed by pulverizing the raw material composition.

Examples of the raw materials corresponding to zinc include zinc oxide, zinc fluoride, and the like. Two or more kinds of zinc raw materials may be used in combination.

Examples of the raw materials corresponding to silicon include anhydrous silicic acid and the like. Two or more kinds of silicon raw materials may be used in combination.

Examples of the raw materials corresponding to aluminum include aluminum oxide, aluminum fluoride, artificial cryolite, and the like. Two or more kinds of aluminum raw materials may be used in combination.

Examples of the raw materials corresponding to calcium include calcium fluoride, calcium phosphate, calcium carbonate, calcium hydroxide, and the like. Two or more kinds of calcium raw materials may be used in combination.

Examples of the raw materials corresponding to fluorine include calcium fluoride, strontium fluoride, sodium fluoride, and the like. Two or more kinds of fluorine raw materials may be used in combination.

Examples of the raw materials corresponding to phosphorus include calcium phosphate, strontium phosphate, sodium dihydrogen phosphate, and the like. Two or more kinds of phosphorus raw materials may be used in combination.

Examples of the raw materials corresponding to boron include boron oxide and the like. Two or more kinds of boron raw materials may be used in combination.

Examples of the raw materials corresponding to sodium include sodium dihydrogen phosphate, sodium carbonate, sodium fluoride, and the like. Two or more kinds of sodium raw materials may be used in combination.

### <Dental Composition>

The dental composition of the present embodiment includes the dental glass powder of the present embodiment.

Examples of the dental compositions in the present embodiment include cement such as a glass ionomer cement, a resin cement, and the like; polymeric compositions such as composite resins and the like; primers; adhesives; and the like. Among them, a glass ionomer cement is preferably used as the dental composition.

### <Glass Ionomer Cement>

Examples of glass ionomer cement include a powder-liquid type glass ionomer cement having a powder component including a dental fluoroaluminosilicate glass powder of the present embodiment, a polycarboxylic acid polymer, and a liquid component that includes water.

The polycarboxylic acid polymer is not particularly limited. For example, a homopolymer or copolymer of α,β-unsaturated carboxylic acid can be used.

Examples of α,β-unsaturated carboxylic acids include acrylic acid, methacrylic acid, 2-chloroacrylic acid, 3-chloroacrylic acid, aconitic acid, mesaconic acid, maleic acid, itaconic acid, fumaric acid, glutaconic acid, citraconic acid, and the like.

The polycarboxylic acid polymer may also be a copolymer of an α,β-unsaturated carboxylic acid and a monomer capable of copolymerizing with an α,β-unsaturated carboxylic acid.

Examples of components capable of copolymerizing with α,β-unsaturated carboxylic acids include acrylamide, acrylonitrile, methacrylate ester, acrylates, vinyl chloride, allyl chloride, vinyl acetate, and the like.

In this case, the ratio of the α,β-unsaturated carboxylic acid to the monomer constituting the polycarboxylic acid polymer is preferably 50% by mass or more.

The polycarboxylic acid polymer is preferably a homopolymer or copolymer of acrylic acid or itaconic acid.

The amount of the polycarboxylic acid polymer in the liquid component is usually 5 to 60% by mass.

The water amount in the liquid component is usually 30 to 70% by mass.

The liquid component may further contain an organic polybasic acid.

Examples of the organic polybasic acids include polycarboxylic acids such as citric acid, malic acid, succinic acid, gluconic acid, and the like; ascorbic acid; and the like.

The amount of the organic polybasic acid in the liquid component is usually 5 to 30% by mass.

At least a portion of the polycarboxylic acid-based polymer may be included as a powder component.

The powder component may further contain a fluoroaluminosilicate glass powder.

The fluoroaluminosilicate glass powder generally includes silicon, aluminum, fluorine, phosphorous, sodium, and strontium.

The amount of silicon oxide (SiO₂) in the fluoroaluminosilicate glass powder is generally 15 to 50% by mass.

The amount of aluminum oxide (Al₂O₃) in the fluoroaluminosilicate glass powder is generally 15 to 35% by mass.

The amount of fluorine (F) in the fluoroaluminosilicate glass powder is generally 1 to 30% by mass.

The amount of phosphorus (V) oxide (P₂O₅) in the fluoroaluminosilicate glass powder is generally 0 to 10% by mass.

The amount of sodium oxide (Na₂O) in the fluoroaluminosilicate glass powder is generally 0 to 15% by mass.

The amount of strontium oxide (SrO) in the fluoroaluminosilicate glass powder is generally 0 to 40% by mass.

The fluoroaluminosilicate glass powder may further contain calcium, potassium, lanthanum, and the like.

The mass ratio of the fluoroaluminosilicate glass powder with respect to the dental fluoroaluminosilicate glass powder in the present embodiment is preferably in the range of 0.3 to 4, and is more preferably in the range of 0.5 to 3. When the mass ratio of the fluoroaluminosilicate glass powder with respect to the dental fluoroaluminosilicate glass powder of the present embodiment is 0.3 or more, the strength of the cured product of the glass ionomer cement is improved. When the mass ratio of the fluoroaluminosilicate glass powder with respect to the dental fluoroaluminosilicate glass powder is 4 or less, the antibacterial effect, transparency, and inhibition of dental decalcification of the cured product of the glass ionomer cement are improved.

Powder-liquid type glass ionomer cement is prepared by kneading the powder and liquid components.

When the powder component and the liquid component are kneaded, the mass ratio of the powder component with respect to the liquid component (hereinafter, referred to as the powder-to-liquid ratio) is preferably 1 to 5, and is more preferably 2.8 to 4.0. When the powder-to-liquid ratio is 1 or more, the compression strength of the cured product of the glass ionomer cement is improved. When the powder-to-liquid ratio is 5 or less, the powder component and the liquid component are easily kneaded.

Other glass ionomer cements include a two-component glass ionomer cement having a first paste containing the dental glass powder of the present embodiment, water, or (meth)acrylate; and a second paste containing a polycarboxylic acid polymer and water.

The two-component glass ionomer cement is prepared by mixing the first and second pastes.

### [Example]

Hereinafter, examples of the present invention will be described, but the present invention is not limited to the examples.

### <Examples 1 to 6, Comparative Examples 1 to 7>

Zinc oxide (ZnO), anhydrous silicic acid (SiO₂), aluminum oxide (Al₂O₃), aluminum fluoride (AlF₃), artificial cryolite (Na₃AlF₆), calcium fluoride (CaF₂), calcium carbonate (CaCO₃), phosphorus oxide (P₂O₅), boron oxide (B₂O₃), sodium fluoride (NaF), strontium fluoride (SrF₂), strontium carbonate (SrCO₃), and lanthanum oxide (La₂O₃) were mixed at a predetermined ratio, and then stirred sufficiently with a mortar to obtain a raw material composition. After the raw composition was placed in a platinum crucible, the platinum crucible was placed in the electric furnace. The electric furnace was heated to 1450°C, the raw material composition was melted and homogenized sufficiently, and then flowed into water to obtain a bulk glass. The bulk glass was milled for 4 hours using an alumina ball mill and then passed through a 120-mesh sieve to obtain a glass powder.

Next, the composition of the glass powder and the number average particle size were evaluated.

### <Composition of Glass Powder>

Using a ZSX Primus II fluorescent X-ray analyzer (manufactured by Rigaku Corporation), the glass powder was analyzed, and the composition of the glass powder was determined.

### <Number Average Particle Size of Glass Powder>

A laser diffraction scattering particle size distributor LA-950 (manufactured by Horiba, Ltd.) was used to measure the number average particle size of glass powder.

Next, the antibacterial effect, transparency, and inhibition of dental decalcification of the cured product of the glass ionomer cement including glass powder were evaluated.

### <Preparation of Kneaded Product of Glass Ionomer Cement>

A powder component was obtained by mixing 40% by mass of the glass powder with 60% by mass of the fluorosilicate glass powder. Here, the composition of the fluorosilicate glass powder is SiO₂ (26.8% by mass), Al₂O₃ (23.9% by mass), F (14.3% by mass), SrO (33.0% by mass), P₂O₅ (1.4% by mass), and Na₂O (0.6% by mass), and the number average particle size is 7.6 µm.

A liquid component was obtained by mixing 40% by mass of polyacrylic acid, 50% by mass of water, and 10% by mass of citric acid.

The powder component and the liquid component were kneaded at a powder-to-liquid ratio of 2.8 to obtain a kneaded product of the glass ionomer cement.

### <Antibacterial Effect>

A mold that is 10 mm in diameter and 2 mm in thickness was filled with the kneaded product of the glass ionomer cement and allowed to stand at 37°C for 1 hour under the environment of 90% relative humidity (RH) to cure the kneaded product of the glass ionomer cement. The cured product was then removed from the mold and immersed in 10 mL of a brain heart infusion (BHI) medium for 24 hours. After removing the cured product from the BHI medium, *Streptococcus mutans* (S. mutans) were seeded to an OD540 value of 0.01 and incubated at 37°C for 24 hours. Next, the OD540 value of the BHI medium cultured with S. mutans was measured to evaluate the antibacterial effect.

Here, the OD540 value indicates the optical density of light at a wavelength of 540 nm. The value was measured using a plate reader, SpectraMax M2 (manufactured by Molecular Device Japan).

The criteria for antibacterial effect are as follows. Here, the lower value of the OD540, the higher the antibacterial effect.

Excellent: The value of OD540 is lower than 0.10.

Good: The value of OD540 is 0.10 or higher and lower than 0.15.

Poor: The value of OD540 is 0.15 or higher.

### <Transparency>

A mold that is 15 mm in diameter and 0.5 mm in thickness was filled with the kneaded product of the glass ionomer cement and allowed to stand at 37°C for 1 hour under the environment of 90% RH to cure the kneaded product of the glass ionomer cement. The cured product was then removed from the mold, and a colorimeter was used to measure the L value on a white background and the L value on a black background. Next, the difference (ΔL) between the L value on the white background and the L value on the black background was calculated.

The transparency criteria are as follows. Here, the greater the value of ΔL, the higher the transparency.

Excellent: The value of ΔL is 13 or greater.

Good: The value of ΔL is 10 or greater and less than 13.

Poor: The value of ΔL is less than 10.

### <Inhibition of Dental Decalcification>

A sample of bovine dentin was polished with water-resistant polish paper #1200 by injecting water and a polytetrafluoroethylene seal with a 3 mm diameter hole was applied to the flattened polished surface. Next, the kneaded product of the glass ionomer cement was applied to one half of the polished surface corresponding to the hole of the seal, and then left in a constant temperature humidity bath at 37°C and 100% RH for 24 hours. The kneaded product of the glass ionomer cement was cured to form a cured product.

The bovine dentin with cured product formed was immersed in a 37°C decalcifying fluid (50 mM acetic acid, 1.5 mM calcium chloride, 0.9 mM potassium dihydrogen phosphate, pH 4.5) for 24 hours. At the time, the cured product was not formed on the other half of the polished surface corresponding to the hole of the seal. The other half of the polished surface with cured product not formed was used as a test surface.

A precision cutting machine was used to cut the bovine dentin with cured product formed so as to have a test piece that is 1 mm thick.

After the test surface of the test piece was photographed using an X-ray apparatus, the image of the test surface was analyzed using image processing software to determine the extent of mineral loss and evaluate the inhibition of dental decalcification.

The criteria for the inhibition of dental decalcification are as follows. Here, the smaller the extent of mineral loss, the higher the inhibition of dental decalcification.
Excellent: The extent of mineral loss is less than 2300% by volume·µm
Good: The extent of mineral loss is 2300% by volume·µm or higher and less than 2600% by volume·µm.
Poor: The extent of mineral loss is 2600% by volume·µm or higher.

Next, when the inhibition of dental decalcification was evaluated in the same manner as above, except that the kneaded product of the glass ionomer cement was not applied, the extent of mineral loss was 4557% by volume·µm or higher.

Table 1 indicates that the evaluation results of the antibacterial effect, transparency, and inhibition of dental decalcification of the cured products of the glass ionomer cements.

**[Table 1]**

| | Examples | | | | | | Comparative Examples | | | | | | |
|---|---|---|---|---|---|---|---|---|---|---|---|---|---|
| | 1 | 2 | 3 | 4 | 5 | 6 | 1 | 2 | 3 | 4 | 5 | 6 | 7 |
| ZnO | 32.5 | 19.8 | 25.0 | 30.2 | 15.1 | 21.5 | 0 | 0 | 40.5 | 10.6 | 15.9 | 24.1 | 34.5 |
| SiO₂ | 40.6 | 41.7 | 47.1 | 37.5 | 50.4 | 45.6 | 29.8 | 25.7 | 41.6 | 50.4 | 26.6 | 28.6 | 40.3 |
| Al₂O₃ | 12.2 | 15.0 | 12.8 | 7.5 | 17.4 | 15.9 | 19.8 | 19.2 | 6.4 | 17.4 | 19.9 | 0 | 6.4 |
| F | 5.9 | 4.7 | 6.1 | 5.4 | 4.8 | 5.7 | 11.0 | 14.1 | 4.8 | 9.3 | 8.1 | 5.3 | 0 |
| CaO | 8.8 | 10.5 | 9.0 | 10.3 | 12.3 | 11.3 | 0 | 0.1 | 4.0 | 12.3 | 0 | 6.7 | 13.6 |
| SrO | 0 | 0 | 0 | 0 | 0 | 0 | 27.0 | 35.6 | 0 | 0 | 23.9 | 0 | 5.2 |
| La₂O₃ | 0 | 0 | 0 | 0 | 0 | 0 | 6.3 | 0 | 0 | 0 | 0 | 35.3 | 0 |
| P₂O₅ | 0 | 0 | 0 | 7.0 | 0 | 0 | 3.6 | 4.1 | 0 | 0 | 3.1 | 0 | 0 |
| Na₂O | 0 | 0 | 0 | 2.1 | 0 | 0 | 2.5 | 1.2 | 2.7 | 0 | 2.5 | 0 | 0 |
| B₂O₃ | 0 | 8.3 | 0 | 0 | 0 | 0 | 0 | 0 | 0 | 0 | 0 | 0 | 0 |
| Total | 100 | 100 | 100 | 100 | 100 | 100 | 100 | 100 | 100 | 100 | 100 | 100 | 100 |
| Number average particle size [µm] | 7.3 | 7.8 | 7.6 | 8.1 | 7.6 | 7.3 | 8.3 | 7.8 | 7.6 | 8.1 | 8.3 | 7.4 | 7.7 |
| Antibacterial effect | Good | Good | Excellent | Excellent | Good | Excellent | Poor | Poor | Excellent | Poor | Good | Excellent | Excellent |
| Transparency | Excellent | Excellent | Excellent | Good | Excellent | Excellent | Excellent | Excellent | Poor | Excellent | Excellent | Poor | Good |
| Inhibition of dental decalcification | Excellent | Excellent | Excellent | Good | Excellent | Excellent | Poor | Poor | Good | Excellent | Poor | Good | Poor |

From Table 1, it can be seen that the glass ionomer cement having the powder components containing the glass powder of Examples 1 to 6 exhibited high antibacterial efficacy, transparency, and inhibition of dental decalcification of the cured products.

In contrast, the glass ionomer cement having the powder component containing the glass powder of Comparative Example 1 exhibited low antibacterial effect and low inhibition of dental decalcification of the cured product, likely because the glass powder of Comparative Example 1 did not contain zinc and calcium.

The glass ionomer cement including the powder component containing the glass powder of Comparative Example 2 showed low antibacterial effect of the cured product and low inhibition of dental decalcification, likely because the glass powder of Comparative Example 2 did not contain zinc, and the amount of calcium oxide (CaO) in the glass powder of Comparative Example 2 was 0.1% by mass.

The glass ionomer cement having the powder component containing the glass powder of Comparative Example 3 showed low transparency of the cured product, likely because the amount of zinc oxide (ZnO) in the glass powder of Comparative Example 3 was 40.5% by mass.

The glass ionomer cement having the powder component containing the glass powder of Comparative Example 4 showed low antibacterial effect of the cured product, likely because the amount of zinc oxide (ZnO) in the glass powder of Comparative Example 4 was 10.6% by mass.

The glass ionomer cement having the powder component containing the glass powder of Comparative Example 5 showed low inhibition of dental decalcification of the cured product, likely because the glass powder of Comparative Example 5 did not contain calcium.

The glass ionomer cement having the powder component containing the glass powder of Comparative Example 6 showed low transparency of the cured product, likely because the glass powder of Comparative Example 6 did not contain aluminum.

The glass ionomer cement having the powder component containing the glass powder of Comparative Example 7 showed low inhibition of dental decalcification of the cured product, likely because the glass powder of Comparative Example 7 did not contain fluorine.

## Claims

1. A dental composition containing a glass powder, the glass powder comprising:
15 to 40% by mass of zinc oxide (ZnO);
20 to 55% by mass of silicon oxide (SiO₂);
6 to 20% by mass of aluminum oxide (Al₂O₃);
1 to 13% by mass of calcium oxide (CaO), and
1 to 19% by mass of fluorine (F),
wherein the dental composition is a glass ionomer cement,
wherein the glass ionomer cement includes a powder component having fluoroaluminosilicate glass powder, a polycarboxylic acid polymer, and a liquid component including water, and
wherein a mass ratio of the powder component to the liquid component is in a range from 1 to 5.

2. The dental composition according to claim 1,
wherein the glass powder further contains boron, and
wherein a content of boron oxide (B₂O₃) is more than 0% by mass and 15% by mass or less.

3. The dental composition according to claim 1 or 2,
wherein the glass powder further contains phosphorus, and
wherein a content of phosphorus(V) oxide (P₂O₅) is more than 0% by mass and 10% by mass or less.

4. The dental composition according to any one of claims 1 to 3,
wherein the glass powder further contains sodium, and
wherein a content of sodium oxide (Na₂O) is more than 0% by mass and 10% by mass or less.

5. The dental composition according to claim 1,
wherein the mass ratio of the powder component to the liquid component is in a range from 2.8 to 4.

## Patentansprüche

1. Dentalzusammensetzung, enthaltend ein Glaspulver, wobei das Glaspulver umfasst:
15 bis 40 Massen-% von Zinkoxid (ZnO);
20 bis 55 Massen-% von Siliziumoxid (SiO₂);
6 bis 20 Massen-% von Aluminiumoxid (Al₂O₃);
1 bis 13 Massen-% von Calciumoxid (CaO), und
1 bis 19 Massen-% von Fluor (F),
wobei die Dentalzusammensetzung ein Glasionomerzement ist,
wobei der Glasionomerzement einen Pulverbestandteil mit Fluoraluminosilicat-Glaspulver, ein Polycarbonsäure-Polymer und einen flüssigen Bestandteil beinhaltend Wasser beinhaltet, und
wobei ein Massenverhältnis des Pulverbestandteils zu dem flüssigen Bestandteil in einem Bereich von 1 bis 5 ist.

2. Dentalzusammensetzung nach Anspruch 1,
wobei das Glaspulver weiter Bor enthält, und
wobei ein Gehalt von Boroxid (B₂O₃) mehr als 0 Massen-% und 15 Massen-% oder weniger beträgt.

3. Dentalzusammensetzung nach Anspruch 1 oder 2,
wobei das Glaspulver weiter Phosphor enthält, und
wobei ein Gehalt von Phosphor(V)-oxid (P₂O₅) mehr als 0 Massen-% und 10 Massen-% oder weniger beträgt.

4. Dentalzusammensetzung nach einem der Ansprüche 1 bis 3,
wobei das Glaspulver weiter Natrium enthält, und
wobei ein Gehalt von Natriumoxid (Na₂O) mehr als 0 Massen-% und 10 Massen-% oder weniger beträgt.

5. Dentalzusammensetzung nach Anspruch 1,
wobei das Massenverhältnis des Pulverbestandteils zu dem flüssigen Bestandteil in einem Bereich von 2,8 bis 4 ist.

## Revendications

1. Composition dentaire contenant une poudre de verre, la poudre de verre comprenant :
15 à 40 % en masse d'oxyde de zinc (ZnO) ;
20 à 55 % en masse d'oxyde de silicium (SiO₂) ;
6 à 20 % en masse d'oxyde d'aluminium (Al₂O₃) ;
1 à 13 % en masse d'oxyde de calcium (CaO), et
1 à 19 % en masse de fluor (F),
dans laquelle la composition dentaire est un ciment de verre ionomère,
dans laquelle le ciment de verre ionomère comporte un composant en poudre ayant une poudre de verre fluoroaluminosilicaté, un polymère d'acide polycarboxylique, et un composant liquide comportant de l'eau, et
dans laquelle un rapport massique du composant en poudre au composant liquide est dans une plage de 1 à 5.

2. Composition dentaire selon la revendication 1,
dans laquelle la poudre de verre contient en outre du bore, et
dans laquelle une teneur d'oxyde de bore (B₂O₃) est supérieure à 0 % en masse et inférieure ou égale à 15 % en masse.

3. Composition dentaire selon la revendication 1 ou 2,
dans laquelle la poudre de verre contient en outre du phosphore, et
dans laquelle une teneur d'oxyde de phosphore (V) (P₂O₅) est supérieure à 0 % en masse et inférieure ou égale à 10 % en masse.

4. Composition dentaire selon l'une quelconque des revendications 1 à 3,
dans laquelle la poudre de verre contient en outre du sodium, et
dans laquelle une teneur d'oxyde de sodium (Na₂O) est supérieure à 0 % en masse et inférieure ou égale à 10 % en masse.

5. Composition dentaire selon la revendication 1,
dans laquelle le rapport massique du composant en poudre au composant liquide est dans une plage de 2,8 à 4.
